# EUROPEAN PATENT APPLICATION

(11) **EP 0 864 571 A1**
(43) Date of publication of application: **16.09.1998**
(21) Application number: 95938607.9
(22) Date of filing: 29.11.1995
(51) Int. Cl.: C07D 461/00, A61K 31/435

(54) **APOVINCAMINIC ACID DERIVATIVE AND MEDICINE CONTAINING THE SAME**

(71) Applicant: Hidaka, Hiroyoshi, Nagoya-shi, Aichi 468 (JP)
(72) Inventor: HIDAKA, Hiroyoshi, Nagoya-shi, Aichi 468 (JP); ISHIKAWA, Tomohiko, Nagoya-shi, Aichi 466 (JP); MURAMATSU, Hiroshi, Hino-shi, Tokyo 191 (JP); INOUE, Tsutomu, Funabashi-shi, Chiba 274 (JP)
(74) Representative: Hansen, Bernd, Dr. Dipl.-Chem.
(86) International application number: JP9502434
(87) International publication number: WO9719945

(57) **Abstract**

This invention relates to an apovincaminic acid derivative represented by the following formula (1): 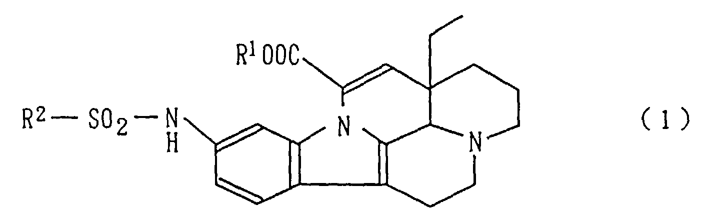 wherein R¹ represents a lower alkyl group, and R² represents an aryl group, which may be substituted by 1 to 5 substituents selected from halogen atoms and lower alkyl, lower alkoxy, lower alkoxycarbonyl, acylamino and nitro groups, a benzyl group, a quinolyl group or a thienyl group, or an acid addition salt thereof; and also to a drug containing the same. This compound has excellent vasodilating activity and platelet aggregation inhibiting activity, and can be used for the treatment of cerebrovascular disorder, thromboembolism and chronic arterial occlusive diseases and also for the improvement of blood flow disturbances.

## Description

### Technical Field

This invention relates to novel apovincaminic acid derivatives, and more specifically to apovincaminic acid derivatives and acid derivatives thereof, which have excellent vasodilating activity and platelet aggregation inhibiting activity and are useful as drugs.

### Background Art

Apovincaminic acid derivatives have already been reported to be effective for the treatment of cerebrovascular disorder, peripheral circulatory disorder, angina pectoris, and hypertension (JP Kokai 56-71091, ibid. 59-62590). In particular, ethyl apovincaminate (vinpocetine) has been subjected to clinical tests.

The conventionally-known ethyl apovincaminate is however still not considered to be sufficient, resulting in a demand for the development of apovincaminic acid derivatives having still better effects.

### Disclosure of the Invention

The present inventors have therefore proceeded with extensive research and, as a result, have found that novel apovincaminic acid derivatives with a sulfonylamino group introduced to the 11-position of the apovincaminic acid skeleton have excellent platelet aggregation inhibiting activity along with superb vasodilating activity and are useful as therapeutics for various circulatory diseases, leading to the completion of the present invention.

Accordingly, the present invention provides an apovincaminic acid derivative represented by the following formula (1): 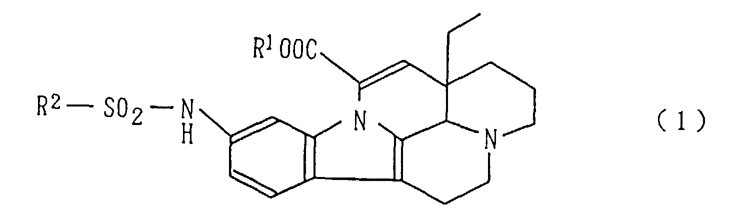 wherein R¹ represents a lower alkyl group, and R² represents an aryl group, which may be substituted by 1 to 5 substituents selected from halogen atoms and lower alkyl, lower alkoxy, lower alkoxycarbonyl, acylamino and nitro groups, a benzyl group, a quinolyl group or a thienyl group; or an acid addition salt thereof.

In addition, this invention also provides a drug comprising an apovincaminic acid derivative represented by the formula (1) or an acid addition salt thereof.

Further, this invention also provides a pharmaceutical preparation comprising an apovincaminic acid derivative represented by the formula (1) or an acid addition salt thereof and a pharmacologically acceptable carrier or diluent.

Moreover, this invention also provides use of an apovincaminic acid derivative represented by the formula (1) or an acid addition salt thereof as a drug.

Furthermore, this invention also provides a therapeutic method of a circulatory disease, which comprises administering an effective amount of an apovincaminic acid derivative represented by the formula (1) or an acid addition salt thereof.

### Best Mode for Carrying Out the Invention

In the formula (1) of this invention, the lower alkyl group may be a linear or branched alkyl group having 1 to 6 carbon atoms, examples of which can include methyl, ethyl, n-propyl, isopropyl, n-butyl, sec-butyl, tert-butyl, n-pentyl, isopentyl, n-hexyl, and isohexyl groups. Illustrative of the halogen atoms can be fluorine, chlorine, bromine and iodine atoms. The lower alkoxy groups may be alkoxy groups having 1 to 6 carbon atoms, examples of which can include methoxy, ethoxy, propoxy and butoxy groups. The lower alkoxycarbonyl groups may be alkoxycarbonyl groups containing 1 to 6 carbon atoms in their alkoxy groups, examples of which can include methoxycarbonyl, ethoxycarbonyl, propoxycarbonyl and butoxycarbonyl groups. The acylamino groups may be acylamino groups containing 1 to 6 carbon atoms in their acyl groups, examples of which can include acetylamino, propionylamino, butyrylamino and valeryl amino groups. Further, illustrative of the aryl group can be phenyl and naphthyl groups. The aryl group may be substituted by desired 1 to 5 substituents selected from halogen atoms and lower alkyl, lower alkoxy, lower alkoxycarbonyl, acylamino and nitro groups.

Examples of the acid addition salt of the apovincaminic acid derivative (1) according to the present invention can include its pharmacologically acceptable salts, for example, its inorganic acid salts such as its hydrochloride, hydrobromide, sulfate, phosphate and nitrate; and its organic acid salts such as its formate, acetate, propionate, glycolate, maleate, fumarate, succinate, tartrate, ascorbate, citrate, malate, salicylate, lactate, benzoate, p-toluenesulfonate and methanesulfonate. Further, the hydrate of the apovincaminic acid derivative (1) or its acid addition salt is also included in the present invention.

The apovincaminic acid derivative (1) according to the present invention can be prepared, for example, by a process indicated by the following reaction scheme. 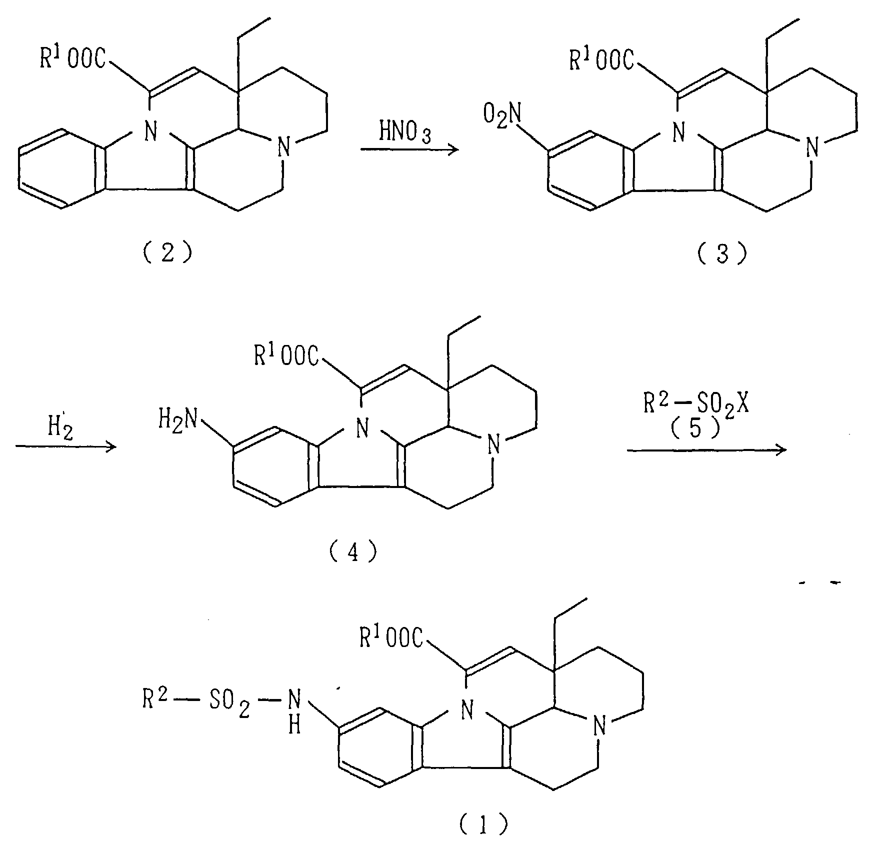 wherein X represents a halogen atom, and R¹ and R² represent the same groups as described above.

Namely, an apovincaminate ester (2) is nitrated into an 11-nitro-apovincaminate ester (3), followed by the reduction into an 11-amino-apovincaminate ester (4). A sulfonyl halogenide (5) is then reacted further, whereby an apovincaminic acid derivative (1) is prepared.

The apovincaminate ester as the raw material can be easily prepared by esterifying apovincaminic acid which is known from JP Kokai 59-62590.

The nitration of the apovincaminate ester (2) may be conducted by the general nitration method which makes use of concentrated nitric acid. When this method is followed, compounds with nitro groups substituted to the 9-, 10- and 11-positions of the apovincamine skeleton, respectively, is obtained as a mixture. They can however be readily separated by column chromatography or the like.

The reduction of the 11-nitro-apovincaminate ester (3) may be effected by catalytic reduction, which makes use of a catalyst such as platinum dioxide, Raney nickel or palladium, or the like.

The reaction between the 11-aminoapovincaminate ester (4) and the sulfonyl halogenide (5) may be conducted in the presence of a base such as pyridine or triethylamine, in an inert solvent such as pyridine, tetrahydrofuran, dimethylformamide or dichloromethane, under ice cooling or at room temperature.

The apovincaminic acid derivative (1) according to the present invention has blood flow enhancing activity based on vasodilation and antithrombotic activity by inhibition of platelet aggregation, so that it can be used as therapeutics for cerebrovascular disorder (post-cerebral infarct, post-intracerebral hemorrhage, cerebral arteriosclerosis, cerebral thrombosis), ischemic heart diseases (angina pectoris, myocardial infarction, etc.), and thromboembolism, chronic arterial occlusive diseases and the like associated with these diseases; as an improver for blood flow disturbances; and also as therapeutics for circulatory diseases, such as a hypotensive agent.

For the above-described purposes, the apovincaminic acid derivative (1) or the acid addition salt thereof according to the present invention can be administered orally or parenterally. With the addition of pharmacologically acceptable carriers or diluents, the apovincaminic acid derivative (1) can therefore be formulated into pharmaceutical preparations such as tablets, powders, granules, capsules, liquids, emulsions, suspensions, external skin care preparations and injections by methods known *per se* in the art. Usable carriers or diluents can include excipients, extenders, binders, humectants, disintegrants, surfactants, lubricants, dispersants, buffers, preservatives, corrigents, perfumes, coating agents, and the like.

It is generally preferred to administer the apovincaminic acid derivative (1) or the acid addition salt thereof at a daily dose of from 1 to 500 mg per adult, although its dose varies depending on the age, the kind and severity of the disease, the administration method and the like.

### Examples

The present invention will next be described in further detail by the following Examples and Tests.

### Example 1

### (i) Synthesis of ethyl apovincaminate

Vincaminic acid (6.0 g) was added to 180 mℓ of ethanol. After 34.5 g (352 mmol) of concentrated sulfuric acid were carefully added under stirring over ice bath, the resultant mixture was heated under reflux for 8 hours. The reaction mixture was concentrated under reduced pressure, followed by the addition of 100 mℓ of ice water. The thus-obtained mixture was neutralized with a 10% aqueous solution of NaOH. Precipitated crystals were extracted with dichloromethane (50 mℓ x 3). The extracts were washed with a saturated aqueous solution of NaCl and then dried over magnesium sulfate. The solvent was distilled off under reduced pressure. Ethanol was added to the residue to induce crystallization. Crystals were collected by filtration, whereby 4.9 g of the title compound were obtained (yield: 79%).
¹H-NMR(270MHz,CDCℓ₃)δ(ppm):
   7.46(1H,d,J=8.6Hz), 7.24(1H,d,J=8.6Hz), 7.18-7.08(2H,m), 6.11(1H,s), 4.47-4.38(2H,m), 4.14(1H,brs), 3.35(1H,dd,J=13.7,5.4Hz), 3.25(1H,ddd,J=13.5,11.2,5.0Hz), 3.09-2.93(1H,m), 2.62(2H,dd,J=8.7,2.8Hz), 2.50(1H,brd,J=16.3Hz), 1.91(2H,m), 1.76-1.63(1H,m), 1.51(1H,brd,J=13.5Hz), 1.43-1.32(1H,m), 1.39(3H,t,J=7.3Hz), 1.01(3H,t,J=7.6Hz), 1.06-0.91(1H,m).

### (ii) Synthesis of ethyl 11-nitro-apovincaminate

To 100 mℓ of acetic anhydride, 100 mℓ of 70% nitric acid were added dropwise under ice cooling and stirring over about 20 minutes, followed by the further addition of 1 droplet of concentrated sulfuric acid. The resulting solution was stirred for about 15 mℓ under the same conditions. To the thus-obtained solution, 40 g of ethyl apovincaminate were added in small portions under the same conditions, and the resulting mixture was stirred for about 2 hours. The reaction mixture was carefully neutralized with a 20% aqueous solution of NaOH and was then extracted with ethyl acetate (200 mℓ x 3). The extracts were washed with a saturated aqueous solution of NaCl and then dried over magnesium sulfate. The solvent was distilled off under reduced pressure. The resulting residue (59 g) was subjected to column chromatography by using 2 kg of silica gel, and elution was then conducted with ethyl acetate which contained 1% of triethylamine, whereby 18 g of the title compound were obtained (yield: 40%).
¹H-NMR(270MHz,CDCℓ₃)δ(ppm):
   8.28(1H,d,J=2.0Hz), 8.05(1H,dd,J=8.6,2.0Hz), 7.49(1H,d,J=8.6Hz), 6.34(1H,S), 4.53-4.44(2H,m), 4.16(1H,brs), 3.39(1H,dd,J=13.8,5.6Hz), 3.25(1H,ddd,J=13.8,11.0,5.1Hz), 3.13-2.98(1H,m), 2.72-2.48(3H,m), 1.94(2H,m), 1.77-1.67(1H,m), 1.57(1H,brd,J=13.5Hz), 1.43(3H,t,J=7.1Hz), 1.46-1.33(1H,m), 1.04(3H,t,J=7.4Hz), 1.06-0.91(1H,m).

### (iii) Synthesis of ethyl 11-amino-apovincaminate

Dissolved in 200 mℓ of ethanol were 3.7 g of ethyl 11-nitro-apovincaminate, followed by the addition of 200 mg of platinum dioxide. Under a hydrogen gas atmosphere, the resulting mixture was stirred at surrounding temperature and pressure for 2 hours. After the completion of the reaction was confirmed by TLC, the reaction mixture was filtered through "Celite". The solvent was distilled off under reduced pressure, whereby the title compound was obtained.
¹H-NMR(270MHz,CDCℓ₃)δ(ppm):
   7.23(1H,dd,J=7.4,1.8Hz), 6.56(1H,s), 6.56(1H,dd,J=7.4,1.8Hz), 6.06(1H,s), 4.42(2H,dd,J=14.2,6.9Hz), 4.09(1H,brs), 3.61(2H,s), 3.32(1H,dd,J=13.5,5.3Hz), 3.21(1H,ddd,J=13.5,10.9,5.0Hz), 3.04-2.91(1H,m), 2.68-2.56(2H,m), 2.43(1H,brd), 1.89(2H,m), 1.77-1.63(1H,m), 1.50(1H,brd,J=13.5Hz), 1.40(3H,t,J=7.1Hz), 1.43-1.35(1H,m), 1.00(3H,t,J=7.4Hz), 1.07-0.90(1H,m).

### (iv) Synthesis of ethyl 11-phenylsulfonylamino-apovin-caminate (Compound 1)

Dissolved in 20 mℓ of pyridine were 500 g of ethyl 11-amino-apovincaminate and, under ice cooling and stirring, 278 mg of benzenesulfonyl chloride were added. The thus-obtained mixture was stirred for 2 hours and a half under the same conditions. A residue, which had been obtained by distilling off the solvent under reduced pressure, was dissolved in methylene chloride. The resulting solution was washed with a saturated aqueous solution of NaCl and the dried over magnesium sulfate. The solvent was distilled off under reduced pressure. The thus-obtained residue was loaded on a column packed with 20 volumes of silica gel, and elution was then conducted with ethyl acetate, whereby 478 mg of the title compound were obtained (yield: 69%).
¹H-NMR(CDCℓ₃)δ(ppm):
   7.78(2H,d,J=6.9Hz), 7.52(1H,t,J=7.4Hz), 7.42(2H,t,J=7.4Hz), 7.24(1H,d,J=8.2Hz), 7.15(1H,d,J=1.7Hz), 6.64(1H,dd,J=8.4,1.8Hz), 6.41(1H,br),6.12(1H,s), 4.52-4.32(2H,m), 4.10(1H,brs), 3.33(1H,dd,J=13.5,5.9Hz), 3.20(1H,ddd,J=13.8,11.1,5.1Hz), 3.03-2.91(1H,m), 2.61-2.53(1H,m), 2.54(1H,dd,J=11.2,2.6Hz), 2.43(1H,dd,J=16.8,3.3Hz), 1.90(2H,m), 1.79-1.68(1H,m), 1.53(1H,brd,J=13.5Hz), 1.44-1.36(1H,m), 1.38(3H,t,J=7.2Hz), 1.03-0.86(1H,m), 1.00(3H,t,J=7.4Hz).

### Examples 2-24

The following compounds were obtained in a similar manner as in Example 1.

### Compound 2: Methyl 11-phenylsulfonylamino-apovincaminate

¹H-NMR(CDCℓ₃)δ(ppm):
   7.76(2H,d,J=6.9Hz), 7.53(1H,t,J=7.4Hz), 7.42(2H,t,J=7.4Hz), 7.25(1H,d,J=8.6Hz), 7.15(1H,d,J=1.7Hz), 6.63(1H,dd,J=8.2,2.0Hz), 6.49(1H,br), 6.16(1H,s), 4.11(1H,brs), 3.93(3H,s), 3.35(1H,dd,J=13.8,6.1Hz), 3.21(1H,ddd,J=13.5,11.2,5.0Hz), 3.03-2.89(1H,m), 2.70-2.54(1H,m), 2.56(1H,dd,J=10.9,2.6Hz), 2.45(1H,dd,J=16.2,2.9Hz), 1.90(2H,m), 1.78-1.62(1H,m), 1.53(1H,brs,J=13.2Hz), 1.44-1.39(1H,m), 1.01(3H,t,J=7.4Hz), 1.03-0.86(1H,m).

### Compound 3: Methyl 11-(4-chlorophenyl)sulfonylamino-apovincaminate

¹H-NMR(CDCℓ₃)δ(ppm):
   7.70(2H,d,J=7.6Hz), 7.36(2H,d,J=7.6Hz), 7.25(1H,d,J=8.3Hz), 7.13(1H,d,J=2.0Hz), 6.68(1H,dd,J=8.3,2.0Hz), 6.17(1H,s), 4.10(1H,brs), 3.94(3H,s), 3.32(1H,dd,J=13.5,5.6Hz), 3.20(1H,ddd,J=13.5,11.2,5.3Hz), 3.04-2.87(1H,m), 2.65-2.59(1H,m), 2.54(1H,dd,J=11.2,2.6Hz), 2.43(1H,ddd,J=16.5,6.2,2.3Hz), 1.89(2H,m), 1.82-1.62(1H,m), 1.51(1H,brd,J=13.5Hz), 1.40(1H,m), 1.00(3H,t,J=7.3Hz), 1.02-0.90(1H,m).

### Compound 4: Ethyl 11-(4-bromophenyl)sulfonylamino-apovincaminate

¹H-NMR(CDCℓ₃)δ(ppm):
   7.64(2H,d,J=8.9Hz), 7.54(2H,d,J=8.9Hz), 7.25(1H,d,J=8.2Hz), 7.14(1H,d,J=2.0Hz), 6.85(1H,br), 6.68(1H,dd,J=8.2,2.0Hz), 6.14(1H,s), 4.50-4.29(2H,m), 4.09(1H,brs), 3.32(1H,dd,J=13.6,5.6Hz), 3.19 (1H,ddd,J=13.6,11.2,5.0Hz), 3.02-2.91(1H,m), 2.59(1H,brs), 2.53(1H,dd,J=11.0,3.0Hz), 2.43(1H,ddd,J=18.2,4.6,2.0Hz), 1.94-1.84(2H,m), 1.79-1.69(1H,m), 1.49(1H,m), 1.38(3H,t,J=7.1Hz), 1.26-1.25(1H,m), 0.99(3H,t,J=7.4Hz), 0.92-0.85(1H,m).

### Compound 5: Isopropyl 11-(4-chlorophenyl)sulfonylamino-apovincaminate

¹H-NMR(CDCℓ₃)δ(ppm):
   7.73(2H,d,J=8.6Hz), 7.39(2H,d,J=8.9Hz), 7.28(1H,d,J=8.9Hz), 7.14(1H,d,J=6.2Hz), 6.70(1H,dd,J=8.2,2.0Hz), 6.58(1H,br), 6.09(1H,s), 5.24(1H,m), 4.09(1H,brs), 3.32(1H,dd,J=13.5,5.6Hz), 3.18(1H,ddd,J=16.2,13.5,5.0Hz), 3.02-2.89(1H,m), 2.60(1H,brs), 2.52(1H,dd,J=10.9,8.3Hz), 2.40(1H,ddd,J=14.2,4.6,2.4Hz), 1.98-1.81(2H,m), 1.78-1.63(1H,m), 1.51(1H,br), 1.41(3H,d,J=6.2Hz), 1.37(3H,d,J=6.2Hz), 1.29-1.22(1H,m), 0.99(3H,t,J=7.3Hz), 0.93-0.85(1H,m).

### Compound 6: Propyl 11-(4-chlorophenyl)sulfonylamino-apovincaminate

¹H-NMR(CDCℓ₃)δ(ppm):
   7.71(2H,d,J=8.5Hz), 7.38(2H,d,J=8.5Hz), 7.26(1H,d,J=5.6Hz), 7.12(1H,brs), 6.85(1H,br), 6.68(1H,dd,J=8.2,1.6Hz), 6.14(1H,s), 4.40-4.21(2H,m), 4.10(1H,s), 3.32(1H,dd,J=13.6,6.0Hz), 3.19(1H,ddd,J=13.6,11.2,5.9Hz), 3.02-2.90(1H,m), 2.60(1H,br), 2.55(1H,dd,J=10.9,8.3Hz), 2.43(1H,dd,J=16.2,2.6Hz), 1.98-1.79(2H,m), 1.77-1.70(2H,m), 1.51(1H,d,J=13.6Hz), 1.41(1H,dd,J=13.2,3.0Hz), 1.25(1H,brs), 1.01(3H,t,J=5.0Hz), 0.98(3H,t,J=5.0Hz), 0.93-0.85(1H,m).

### Compound 7: Ethyl 11-(4-fluorophenyl)sulfonylamino-apovincaminate

¹H-NMR(CDCℓ₃)δ(ppm):
   7.82-7.73(2H,m), 7.26(1H,d,J=8.3Hz), 7.15(1H,d,J=2.0Hz), 7.09(2H,t,J=8.9Hz), 6.65(1H,dd,J=8.3,2.0Hz), 6.53(1H,br), 6.14(1H,s), 4.51-4.33(2H,m), 4.10(1H,brs), 3.33(1H,dd,J=13.5,5.9Hz), 3.19(1H,ddd,J=13.5,11.2,5.0Hz), 3.03-2.90(1H,m), 2.60-2.51(2H,m), 2.44(1H,ddd,J=16.5,6.2,2.0Hz), 1.90(2H,m), 1.76-1.61(1H,m), 1.52(1H,brd,J=13.5Hz), 1.40(3H,t,J=7.0Hz), 1.42-1.32(1H,m), 1.00(3H,t,J=7.4Hz), 0.99-0.89(1H,m).

### Compound 8: Ethyl 11-(4-iodophenyl)sulfonylamino-apovincaminate

¹H-NMR(CDCℓ₃)δ(ppm):
   7.77(2H,d,J=8.9Hz), 7.50(2H,d,J=8.9Hz), 7.28(1H,s), 7.14(1H,d,J=2.0Hz), 6.65(1H,d,J=2.0Hz), 6.41(1H,brs), 6.14(1H,s), 4.51-4.35(2H,m), 4.11(1H,brs), 3.33(1H,dd,J=5.6,3.6Hz), 3.20(1H,ddd,J=13.5,11.3,5.3Hz), 3.05-2.89(1H,m), 2.61(1H,brs), 2.57-2.53(1H,m), 2.46-2.41(1H,m), 1.95-1.82(2H,m), 1.57(2H,brs), 1.39(3H,t,J=7.2Hz), 1.28-1.26(1H,m), 1.00(3H,t,J=7.2Hz), 0.93-0.92(1H,m).

### Compound 9: Ethyl 11-(2,5-dichlorophenyl)sulfonylamino-apovincaminate

¹H-NMR(CDCℓ₃)δ(ppm):
   7.96(1H,d,J=2.3Hz), 7.45(1H,d,J=8.6Hz), 7.40 (1H,dd,J=8.6,2.3Hz), 7.26 (1H,d,J=8.2Hz), 7.15(1H,d,J=2.0Hz), 7.00(1H,br), 6.81(1H,dd,J=8.3,2.0Hz), 6.13(1H,s), 4.57-4.37(2H,m), 4.07(1H,brs), 3.31(1H,dd,J=13.8,5.9Hz), 3.18(1H,ddd,J=13.8,11.5,5.6Hz), 3.02-2.86(1H,m), 2.63-2.55(1H,m), 2.54(1H,dd,J=11.2,2.6Hz), 2.42(1H,dd,J=15.8,2.0Hz), 1.89(2H,m), 1.78-1.62(1H,m), 1.59-1.51(1H,m), 1.45(3H,t,J=7.1Hz), 1.47-1.36(1H,m), 0.99(3H,t,J=7.4Hz), 1.02-0.92(1H,m).

### Compound 10: Ethyl 11-(1-naphthalene)sulfonylamino-apovincaminate

¹H-NMR(CDCℓ₃)δ(ppm):
   8.74(1H,d,J=8.9Hz), 8.14(1H,dd,J=7.6,1.3Hz), 8.01(1H,d,J=8.3Hz), 7.93(1H,dd,J=8.3,1.3Hz), 7.70-7.56(2H,m), 7.41(1H,dd,J=8.3,7.6Hz), 7.12(1H,d,J=8.3Hz), 6.93(1H,d,J=1.6Hz), 6.85(1H,br), 6.52(1H,dd,J=8.3,1.6Hz), 6.06(1H,s), 4.38-4.08(2H,m), 4.04(1H,brs), 3.28(1H,dd,J=13.5,5.6Hz), 3.15(1H,ddd,J=13.5,11.0,5.3Hz), 2.97-2.83(1H,m), 2.56(1H,dd,J=5.2,2.6Hz), 2.49(1H,dd,J=11.2,2.6Hz), 2.36(1H,ddd,J=16.5,6.2,2.0Hz), 1.86(2H,m), 1.75-1.59(1H,m), 1.47(1H,brd,J=13.5Hz), 1.42-1.33(1H,m), 1.29(3H,t,J=7.2Hz), 0.97(3H,t,J=7.3Hz), 0.98-0.85(1H,m).

### Compound 11: Ethyl 11-(4-toluene)sulfonylamino-apovincaminate

¹H-NMR(CDCℓ₃)δ(ppm):
   7.66(2H,d,J=8.6Hz), 7.25(1H,d,J=9.2Hz), 7.21(2H,d,J=9.2Hz), 7.15(1H,d,J=1.7Hz), 6.64(1H,dd,J=8.4,1.8Hz), 6.42(1H,br), 6.12(1H,s), 4.52-4.32(2H,m), 4.11(1H,brs), 3.33(1H,dd,J=13.6,5.6Hz), 3.20(1H,ddd,J=13.5,11.2,5.1Hz), 3.03-2.91(1H,m), 2.68-2.53 (1H,m), 2.54 (1H,dd,J=11.2,2.6Hz), 2.43(1H,dd,J=16.5,3.0Hz), 2.37(3H,s), 1.90(2H,m), 1.78-1.63(1H,m), 1.52(1H,brd,J=13.5Hz), 1.38(3H,t,J=7.3Hz), 1.43-1.32(1H,m), 1.00(3H,t,J=7.6Hz), 1.03-0.91(1H,m).

### Compound 12: Ethyl 11-mesytylenesulfonylamino-apovincaminate

¹H-NMR(CDCℓ₃)δ(ppm):
   7.23(1H,d,J=8.6Hz), 7.01(1H,d,J=1.7Hz), 6.91(2H,s), 6.63(1H,dd,J=8.4,1.8Hz), 6.45(1H,br), 6.11(1H,s), 4.45-4.31(2H,m), 4.08(1H,brs), 3.33(1H,dd,J=13.6,5.6Hz), 3.20(1H,ddd,J=13.5,11.2,5.1Hz), 3.03-2.88(1H,m), 2.75-2.62(1H,m), 2.57(6H,s), 2.57-2.49(1H,m), 2.48-2.30(1H,m), 2.27(3H,s), 1.89(2H,m), 1.77-1.63(1H,m), 1.58-1.47(1H,m), 1.38(3H,t,J=7.2Hz), 1.48-1.35(1H,m), 0.99(3H,t,J=7.4Hz), 1.02-0.88(1H,m).

### Compound 13: Ethyl 11-benzylsulfonylamino-apovincaminate

¹H-NMR(CDCℓ₃)δ(ppm):
   7.39(1H,d,J=8.2Hz), 7.34(5H,s), 7.28(1H,d,J=2.0Hz), 6.89(1H,dd,J=8.2,2.0Hz), 6.73(1H,br), 6.17(1H,s), 4.55-4.33(2H,m), 4.33(2H,s), 4.13(1H,brs), 3.32(1H,dd,J=13.5,5.6Hz), 3.22(1H,ddd,J=13.5,5.2,2.6Hz), 3.06-2.95(1H,m), 2.67-2.63(1H,m), 2.58(1H,dd,J=11.2,2.6Hz), 2.48(1H,ddd,J=16.5,6.2,2.0Hz), 1.92(2H,m), 1.83-1.64(1H,m), 1.54(1H,brd,J=13.5Hz), 1.45-1.37(1H,m), 1.40(3H,t,J=7.3Hz), 1.08-0.98(1H,m), 1.01(3H,t,J=7.3Hz).

### Compound 14: Ethyl 11-(4-acetylaminophenyl)sulfonyl-amino-apovincaminate

¹H-NMR(CDCℓ₃)δ(ppm):
   8.12(1H,brs), 7.60(1H,br), 7.59(2H,d,J=8.6Hz), 7.43(2H,d,J=8.6Hz), 7.19(1H,d,J=2.0Hz), 7.13(1H,d,J=8.3Hz), 6.62(1H,dd,J=8.3,2.0Hz), 6.10(1H,s), 4.51-4.28(2H,m), 4.05(1H,brs), 3.28(1H,dd,J=13.5,5.6Hz), 3.15(1H,ddd,J=13.5,11.2,5.3Hz), 2.98-2.82(1H,m), 2.60-2.55(1H,m), 2.51(1H,dd,J=11.2,2.6Hz), 2.46(1H,ddd,J=16.5,6.2,2.0Hz), 2.10(3H,s), 1.87(2H,m), 1.80-1.60(1H,m), 1.48(1H,brd,J=13.2Hz), 1.35(3H,t,J=7.2Hz), 1.40-1.32(1H,m), 0.98(3H,t,J=7.3Hz), 1.00-0.89(1H,m).

### Compound 15: Ethyl 11-(8-quinoline)sulfonylamino-apovincaminate

¹H-NMR(CDCℓ₃)δ(ppm):
   9.17(1H,dd,J=4.3,1.7Hz), 8.40(1H,brs), 8.33-8.28(2H,m), 8.00(1H,dd,J=8.3,1.3Hz), 7.62(1H,dd,J=8.3,4.3Hz), 7.54(1H,dd,J=7.3,8.3Hz), 7.11(1H,d,J=8.3Hz), 7.03(1H,d,J=2.0Hz), 6.62(1H,dd,J=8.3,2.0Hz), 6.04(1H,s), 4.46-4.23(2H,m), 4.02(1H,brs), 3.27(1H,dd,J=13.5,5.7Hz), 3.11(1H,ddd,J=13.5,11.2,5.3Hz), 2.95-2.81(1H,m), 2.59-2.45(2H,m), 2.34(1H,ddd,J=16.2,6.2,2.6Hz), 1.85(2H,m), 1.73-1.64(1H,m), 1.47(1H,brd,J=13.9Hz), 1.39(3H,t,J=7.1Hz), 1.42-1.31(1H,m), 0.96(3H,t,J=7.6Hz), 1.01-0.90(1H,m).

### Compound 16: Ethyl 11-(4-methoxyphenyl) sulfonylamino-apovincaminate

¹H-NMR(CDCℓ₃)δ(ppm):
   7.71(2H,d,J=8.9Hz), 7.25(1H,d,J=6.9Hz), 7.15(1H,d,J=1.7Hz), 6.88(2H,d,J=8.9Hz), 6.66(1H,dd,J=8.3,2.0Hz, 6.43(1H,brs), 6.12(1H,s), 4.53-4.33(2H,m), 4.14(1H,brs), 3.81(3H,s), 3.35(1H,dd,J=13.8,5.9Hz), 3.22(1H,ddd,J=13.8,11.2,5.6Hz), 3.04-2.88(1H,m), 2.72-2.62 (1H,m), 2.58 (1H,dd,J=11.2,2.6Hz), 2.48(1H,dd,J=16.2,2.9Hz), 1.92(2H,m), 1.78-1.60(1H,m), 1.53(1H,brd,J=13.5Hz), 1.39(3H,t,J=7.3Hz), 1.45-1.36(1H,m), 1.01(3H,t,J=7.6Hz), 1.04-0.88(1H,m).

### Compound 17: Ethyl 11-(2,5-dimethylphenyl)sulfonyl-amino-apovincaminate

¹H-NMR(CDCℓ₃)δ(ppm):
   7.86(1H,d,J=1.3Hz), 7.21(1H,d,J=8.3Hz), 7.15(1H,br), 7.16(1H,dd,J=8.0,1.3Hz), 7.13(1H,d,J=8.0Hz), 7.05(1H,d,J=2.0Hz), 6.70(1H,dd,J=8.3,2.0Hz), 6.09(1H,s), 4.51-4.25(2H,m), 4.08(1H,brs), 3.31(1H,dd,J=13.5,5.9Hz), 3.18(1H,ddd,J=13.5,11.2,5.3Hz), 2.99-2.84(1H,m), 2.60(3H,s), 2.63-2.56(1H,m), 2.51(1H,dd,J=11.2,2.7Hz), 2.40(1H,ddd,J=16.5,6.2,2.0Hz), 2.29(3H,s), 1.88(2H,m), 1.78-1.60(1H,m), 1.49(1H,brd,J=13.5Hz), 1.43-1.34(1H,m), 1.37(3H,t,J=7.2Hz), 0.98(3H,t,J=7.3Hz), 1.01-0.87(1H,m).

### Compound 18: Ethyl 11-(2-thiophene)sulfonylamino-apovincaminate

¹H-NMR(CDCℓ₃)δ(ppm):
   7.52-7.49(2H,m), 7.29(1H,d,J=8.3Hz), 7.21(1H,d,J=2.0Hz), 6.98(1H,t,J=4.3Hz), 6.73(1H,dd,J=8.3,2.0Hz), 6.68(1H,br), 6.13(1H,s), 4.54-4.31(2H,m), 4.14(1H,brs), 3.36(1H,dd,J=13.5,5.9Hz), 3.23(1H,ddd,J=13.5,11.2,5.3Hz), 3.05-2.89(1H,m), 2.72-2.62(1H,m), 2.58(1H,dd,J=11.5,2.6Hz), 2.48(1H,dd,J=16.5,2.0Hz), 1.92(2H,m), 1.78-1.68(1H,m), 1.53(1H,brd,J=13.5Hz), 1.39(3H,t,J=7.3Hz), 1.45-1.36(1H,m), 1.01(3H,t,J=7.4Hz), 1.03-0.86(1H,m).

### Compound 19: Ethyl 11-(2-methoxycarbonylphenyl)sulfonylamino-apovincaminate

¹HNMR(CDCℓ₃)δ(ppm):
   7.95(1H,brs), 7.83(1H,dd,J=7.6,1.5Hz), 7.81(1H,dd,J=7.6,1.5Hz), 7.55(1H,dt,J=7.6,1.5Hz), 7.44(1H,dt,J=7.6,1.5Hz), 7.21(1H,d,J=8.2Hz), 7.18(1H,d,J=1.5Hz), 6.77(1H,dd,J=8.2,1.5Hz), 6.08(1H,s), 4.54-4.32(2H,m), 4.08(1H,brs), 4.05(3H,s), 3.32(1H,dd,J=13.8,5.7Hz), 3.17(1H,ddd,J=13.8,11.2,5.3Hz), 3.01-2.90(1H,m), 2.60-2.55(2H,m), 2.41(1H,ddd,J=16.3,6.2,2.8Hz), 1.89(2H,m), 1.74-1.62(1H,m), 1.51(1H,brd,J=13.9Hz), 1.40(3H,t,J=7.3Hz), 1.42-1.37(1H,m), 1.00(3H,t,J=7.5Hz), 1.02-0.95(1H,m).

### Compound 20: Ethyl 11-(2,4,6-triisopropylphenyl)sulfonylamino-apovincaminate

¹H-NMR(CDCℓ₃)δ(ppm):
   7.27(1H,d,J=8.3Hz), 7.09(2H,s), 7.00(1H,d,J=2.0Hz), 6.75(1H,dd,J=8.3,2.0Hz), 6.58(1H,brs), 6.13(1H,s), 4.42-4.24(2H,m), 4.10(1H,brs), 3.96(2H,m), 3.33(1H,dd,J=13.5,5.6Hz), 3.21(1H,ddd,J=13.5,11.2,5.0Hz), 3.02-2.90(1H,m), 2.87(1H,m), 2.60(1H,dd,J=5.2,2.6Hz), 2.53(1H,dd,J=11.2,2.6Hz), 2.44(1H,ddd,J=16.5,6.2,2.0Hz), 1.89(2H,m), 1.64-2.00(1H,m), 1.49(1H,brd,J=13.9Hz), 1.41-1.31(1H,m), 1.34(3H,t,J=7.3Hz), 1.23(6H,d,J=6.9Hz), 1.13(6H,d,J=6.9Hz), 1.09(6H,d,J=6.9Hz), 0.99(3H,t,J=7.3Hz), 0.95-0.83(1H,m).

### Compound 21: Ethyl 11-(4-methoxy-2-nitrophenyl)sulfonylamino-apovincaminate

¹H-NMR(CDCℓ₃)δ(ppm):
   7.78(1H,d,J=8.9Hz), 7.32(1H,d,J=2.5Hz), 7.28(1H,d,J=8.3Hz), 7.21(1H,d,J=2.0Hz), 7.13(1H,br), 6.94(1H,dd,J=8.9,2.5Hz), 6.85(1H,dd,J=8.3,2.0Hz), 6.12(1H,s), 4.52-4.31(2H,m), 4.09(1H,brs), 3.87(3H,s), 3.33(1H,dd,J=13.5,5.6Hz), 3.18(1H,ddd,J=13.5,11.2,5.1Hz), 3.02-2.91(1H,m), 2.65-2.52(2H,m), 2.42(1H,ddd,J=16.2,6.2,2.3Hz), 1.89(2H,m), 1.78-1.62(1H,m), 1.52(1H,brd,J=13.2Hz), 1.41(3H,t,J=7.1Hz), 1.45-1.36(1H,m), 1.00(3H,t,J=7.4Hz), 1.03-0.95(1H,m).

### Compound 22: Ethyl 11-(2-naphthalene)sulfonylamino-apovincaminate

¹H-NMR(CDCℓ₃)δ(ppm):
   8.41(1H,brs), 7.89-7.75(6H,m), 7.61-7.50(2H,m), 7.20(1H,d,J=8.3Hz), 7.19(1H,d,J=2.0Hz), 6.70(1H,dd,J=8.3,2.0Hz), 6.09(1H,s), 4.44-4.20(2H,m), 4.07(1H,brs), 3.30(1H,dd,J=13.5,5.9Hz), 3.17(1H,ddd,J=13.5,11.2,5.2Hz), 2.97-2.84(1H,m), 2.62-2.55(1H,m), 2.53(1H,dd,J=11.2,2.6Hz), 2.39(1H,ddd,J=16.5,6.2,2.0Hz), 1.88(2H,m), 1.78-1.60(1H,m), 1.49(1H,brd,J=13.5Hz), 1.37(1H,m), 1.32(3H,t,J=7.3Hz), 0.98(3H,t,J=7.3Hz), 1.01-0.88(1H,m).

### Compound 23: Ethyl 11-(2,3,4,5,6-pentafluorophenyl)sulfonylamino-apovincaminate

¹H-NMR(CDCℓ₃)δ(ppm):
   7.35(1H,d,J=8.3Hz), 7.03(1H,d,J=2.0Hz), 6.97(1H,dd,J=8.3,2.0Hz), 6.19(1H,s), 4.88(1H,br), 4.50-4.33(2H,m), 4.07(1H,brs), 3.33(1H,dd,J=13.5,5.6Hz), 3.19(1H,ddd,J=13.5,11.2,5.3Hz), 3.03-2.91(1H,m), 2.61(1H,dd,J=5.2,2.6Hz), 2.54(1H,dd,J=11.2,2.6Hz), 2.45(1H,ddd,J=16.5,6.2,2.0Hz), 1.89(2H,m), 1.78-1.62(1H,m), 1.51(1H,brd,J=13.9Hz), 1.42(3H,t,J=7.0Hz), 1.45-1.36(1H,m), 0.99(3H,t,J=7.3Hz), 1.02-0.89(1H,m).

### Compound 24: Ethyl 11-(3-nitrophenyl)sulfonylamno-apovincaminate

¹H-NMR(CDCℓ₃)δ(ppm): (no distinct signal was observed corresponding to NH) 8.66(1H,dd,J=2.0,1.6Hz), 8.33(1H,ddd,J=8.3,2.0,1.0Hz), 8.06(1H,ddd,J=8.2,1.6,1.0Hz), 7.59(1H,dd,J=8.3,8.2Hz), 7.25(1H,d,J=8.3Hz), 7.09(1H,d,J=2.0Hz), 6.75(1H,dd,J=8.3,2.0Hz), 6.15(1H,s), 4.49-4.27(2H,m), 4.09(1H,brs), 3.32(1H,dd,J=13.5,5.6Hz), 3.20(1H,ddd,J=13.5,11.2,5.3Hz), 3.03-2.87(1H,m), 2.66-2.59(1H,m), 2.54(1H,dd,J=11.2,2.6Hz), 2.44(1H,ddd,J=16.5,6.2,2.0Hz), 1.89(2H,m), 1.80-1.62 (1H,m), 1.51(1H,brd,J=13.5Hz), 1.42-1.35(1H,m), 1.38(3H,t,J=7.2Hz), 0.99(3H,t,J=7.3Hz), 1.01-0.89(1H,m).

### Test 1

### Acute toxicity

A test was conducted using male S1C:ICR mice, which were 6 to 7 weeks old and had body weights of from 18 to 36 g, in groups each which consisted of 3 mice.

Predetermined amounts of compounds according to the present invention and a comparative drug (vinpocetine phosphate), each of which had been dissolved in a 5% aqueous solution of glucose, were administered as single shots into the caudal veins of the mice, and the mice were then observed to determine whether they remained under normal conditions or died. Upon expiration of an observation period, their approximate LD₅₀ values were calculated by the provit method. The results are presented in Table 1.

**Table 1**

| Compound | LD₅₀ |
|---|---|
| Compound 1 | 85 mg/kg |
| Compound 11 | 125 mg/kg |
| Compound 12 | 50 mg/kg |
| Vinpocetine phosphate | 60 mg/kg |

### Test 2

### Vascular smooth muscle relaxing activity

Each rabbit was sacrificed under exsanguination, and an incision was then made in the flank thereof. The superior mesenteric artery so isolated was helically cut open into a preparation cut in a helical form by a method known *per se*d in the art. The preparation cut was then suspended under tension in the Krebs-Henseleit solution of 37 ± 0.5°C through which oxygen gas containing 5% of carbon dioxide gas was ebulated. After this preparation cut was caused to contract with potassium chloride to maintain it under predetermined tension, a given test compound was added cumulatively. Relaxing activity was expressed in terms of a concentration (ED₅₀; µM) for 50% relaxation under the assumption that the contractive tension caused with potassium chloride was 100%. The results are presented in Table 2.

### Test 3

### Platelet aggregation inhibiting activity

### Preparation of washed platelets (by the centrifugal washing method)

Blood, which had been collected from a normal volunteer and had immediately been mixed with 1/10 volume of a 0.38% agueous solution of sodium citrate, was converted into a platelet-rich plasma (PRP) by centrifugation (700 x G, 10 minutes). To the PRP, 1/6 volume of the ACD solution (2.2% sodium citrate, 0.8% citric acid, 2.2% glucose - prepared before use) was added, followed by centrifugation (1,500 x G, 10 minutes). A platelet pellet so obtained was suspended in a modified HEPES Tyrode solution (135 mM sodium chloride, 2.7 mM potassium chloride, 1 mM magnesium chloride, 0.1 mg/mℓ glucose, 20 mM HEPE; pH 7.4). After 1/6 volume of the ACD solution was added to the suspension, further centrifugation was conducted (1,500 x G, 5 minutes). A platelet pellet so obtained was suspended in the modified HEPES Tyrode solution, whereby a washed platelet suspension containing about 300,000 platelets/µℓ therein was obtained.

### Measurement of platelet aggregation reaction (by the nephelometric analysis)

Into 270-µℓ aliquots of the washed platelet suspension obtained above, 3-µℓ aliquots of solutions of a given test compound at varied concentrations in an appropriate solvent were added, respectively. The resulting mixtures were each preheated at 37°C for 2 minutes and, subsequent to the addition of 30 µℓ of a 20 µg/mℓ solution of collagen, an absorbance was measured by a 4-channel aggregometer ("HEMA Tracer 601"; manufactured by Nicoh Bioscience Co., Ltd.).

### Determination of effects of the test compounds

As a control, an absorption at the time of maximum aggregation by collagen in the presence of a solvent making use of a given test compound was assumed to be 100 whereas an absorbance before the addition of collagen was assumed to be 0. An inhibition rate (%) was then calculated from an absorbance at the time of maximum aggregation by collagen when the given test compound was added, and a concentration (µM) of the given test compound which achieves 50% inhibition was expressed as "IC₅₀". The results are presented in Table 2.

**Table 2**

| Test compound | Vascular smooth muscle relaxing activity (ED₅₀) | Platelet aggregation inhibiting activity (IC₅₀) |
|---|---|---|
| Compound 1 | 0.6 | 0.23 |
| Compound 2 | 2.1 | 0.38 |
| Compound 3 | 1.7 | 0.25 |
| Compound 4 | 1.0 | 0.76 |
| Compound 5 | 1.3 | 0.94 |
| Compound 6 | 1.3 | 0.96 |
| Compound 7 | 2.0 | 0.91 |
| Compound 8 | 1.3 | 0.81 |
| Compound 9 | 0.9 | 0.88 |
| Compound 10 | 2.0 | 9.20 |
| Compound 11 | 1.0 | 0.05 |
| Compound 12 | 0.2 | 0.05 |
| Compound 13 | 0.3 | 4.40 |
| Compound 14 | 1.2 | 0.47 |
| Compound 15 | 0.5 | 4.10 |
| Compound 16 | 0.7 | 1.00 |
| Compound 17 | 0.71 | 0.72 |
| Compound 18 | 0.63 | 0.40 |
| Compound 19 | 0.2 | 1.80 |
| Compound 20 | 0.93 | - |
| Compound 21 | 2.1 | 0.40 |
| Compound 22 | 0.86 | 0.28 |
| Compound 23 | 1.2 | 0.58 |
| Compound 24 | 0.2 | 0.90 |
| Vinpocetine phosphate | 2.0 | 78.00 |

### Test 4

### Blood flow enhancement test

A test was conducted using male beagles, which were 9 to 13 months old, had body weights of from 9 to 10 kg and had been anesthetized with pentobarbital sodium (25-30 mg/kg), in groups each of which consisted of four beagles. Into one of the femoral veins of each of the beagles, a given test compound in a form dissolved in a 5% agueous solution of glucose was administered at a dose of from 0.1 to 1.0 mg/kg. Vertebral blood flow and femoral blood flow were measured using an electromagnetic flow probe connected to a magnetic flow meter. The results are presented in Table 3.

**Table 3**

| Test compound | Dose (mg/kg) | Bloodflow increment (%) | |
|---|---|---|---|
| | | Vertebral artery | Femoral artery |
| Compound 1 | 0.1 | 2.5 | 6.6 |
| | 0.3 | 23.9 | 5.0 |
| | 1.0 | 66.6 | 7.0 |
| Compound 11 | 0.1 | 5.4 | 2.9 |
| | 0.3 | 11.8 | 5.1 |
| | 1.0 | 32.5 | 4.5 |
| Compound 12 | 0.1 | 0.0 | 0.8 |
| | 0.3 | 8.5 | 0.0 |
| | 1.0 | 26.7 | 4.7 |
| Vinpocetine phosphate | 0.1 | 4.5 | 2.7 |
| | 0.3 | 25.9 | 7.2 |
| | 1.0 | 55.8 | 2.9 |

### Test 5

### Platelet aggregation inhibition test

A test was conducted using male S1c:Hartley guinea pigs, which were 5 to 7 weeks old and had body weights of from 274 to 554 g, in groups each of which consisted of five guinea pigs. A given test compound in a form dissolved in a 5% agueous solution of glucose was orally administered at a dose of 100 mg/kg. One hour after the administration, blood was collected from the abdominal aorta, and a platelet-rich plasma (PRP) was prepared. Maximum aggregation upon addition of collagen (2.5 µg/mg) or arachidonic acid (0.1 mM) as a platelet aggregation inducer to the PRP was measured, and an inhibition rate (%) was then calculated. The results are presented in Table 4.

**Table 4**

| Test compound | Dose (mg/kg) | Platelet aggregation inhibition rate (%) | |
|---|---|---|---|
| | | Collagen | Arachidonic acid |
| Compound 1 | 100 | 85.4 | 45.2 |
| Compound 11 | 100 | 49.1 | 33.9 |
| Compound 12 | 100 | 13.0 | - |
| Vinpocetine phosphate | 100 | - | - |
| Cilostazol | 100 | 12.4 | - |
| | 300 | 74.7 | 22.1 |

It is appreciated from Tests 1-4 that each compound according to the present invention has excellent vasodilating activity and platelet aggregation inhibiting activity.

### Capability of Exploitation in Industry

The apovincaminic acid derivatives (1) and their acid addition salts according to the present invention have excellent vasodilating activity and platelet aggregation inhibiting activity, and can be used as therapeutics for circulatory diseases, for example, for the treatment of cerebrovascular disorders, ischemic heart diseases, thromboembolism and chronic arterial occlusive diseases and also for the improvement of blood flow disturbances.

## Claims

1. An apovincaminic acid derivative represented by the following formula (1): 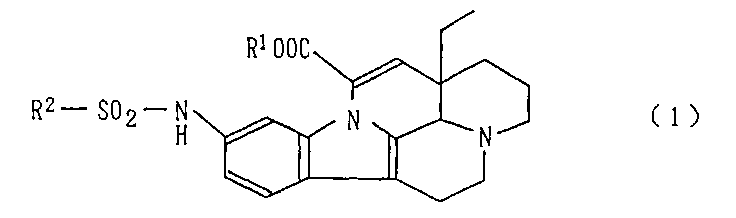 wherein R¹ represents a lower alkyl group, and R² represents an aryl group, which may be substituted by 1 to 5 substituents selected from halogen atoms and lower alkyl, lower alkoxy, lower alkoxycarbonyl, acylamino and nitro groups, a benzyl group, a quinolyl group or a thienyl group; or an acid addition salt thereof.

2. A drug comprising as an effective ingredient the apovincaminic acid derivative or the acid addition salt thereof according to claim 1.

3. A drug according to claim 2, which is a therapeutic for a circulatory disease.

4. A pharmaceutical preparation comprising the apovincaminic acid derivative or the acid addition salt according to claim 1 and a pharmacologically acceptable carrier or diluent.

5. Use of the apovincaminic acid derivative or the acid addition salt according to claim 1 as a drug.

6. Use according to claim 5, wherein said drug is a therapeutic for a circulatory disease.

7. A therapeutic method for a circulatory disease, which comprises administering an effective amount of the apovincaminic acid derivative or the acid addition salt according to claim 1.
